# EUROPEAN PATENT APPLICATION

(11) **EP 4 378 473 A1**
(43) Date of publication of application: **05.06.2024**
(21) Application number: 22849791.3
(22) Date of filing: 20.07.2022
(51) Int. Cl.: A61K 39/00, A61P 35/00

(54) **HER2 VACCINE COMPOSITION**

(30) Priority: 28.07.2021 KR 20210099411
(71) Applicant: Aston Sci. Inc., Seoul 06193 (KR)
(72) Inventor: JUNG, Hun, Seoul 05572 (KR); PARK, Hyo Hyun, Seoul 05229 (KR)
(74) Representative: ZBM Patents - Zea, Barlocci & Markvardsen
(86) International application number: PCT/KR2022/010597
(87) International publication number: WO 2023/008815

(57) **Abstract**

The present invention relates to an HER2-ICD DNA vaccine composition. The vaccine composition according to the present invention can effectively inhibit growth of gastric cancer without serious side effects in an animal model transplanted with a human gastric cancer cell line that expresses HER2, and thus may be usefully used in treatment of gastric cancer.

## Description

### TECHNICAL FIELD

This application claims the benefit of Korean Patent Application No. 1 0-2021-0099411, filed on July 28, 2021, in the Korean Intellectual Property Office, the disclosure of which is incorporated herein in its entirety by reference.

The present invention relates to an HER2 vaccine composition for treating a gastric cancer.

### BACKGROUND ART

gastric cancer refers to all types of cancer occurring in the stomach in principle, and may occur in various forms, such as adenocarcinoma which occurs mainly in the gastric mucous membrane, gastrointestinal tumor which occurs in stromal cells of the stomach, and the like. As with other carcinomas, gastric cancer may be spread to other organs including the esophagus, lung, and liver. Gastric cancer is the 4th most commonly diagnosed cancer in the world in terms of diagnosis frequency, and 930,000 cases have been diagnosed with gastric cancer in 2002. High incidence of gastric cancer leads to high mortality (about 800,000 cases per year), and gastric cancer is the second most common cause of cancer death in the world after lung cancer. This cancer occurs more often in men than in women, and occurs more frequently in Asian and developing countries.

Treatment options for gastric cancer includes surgery, chemotherapy, radiotherapy, or concurrent chemoradiotherapy. Surgery is the first-line therapy for gastric cancer. A 5-year survival rate of surgical resection for the therapeutic purpose appears to be in a range of about 30 % to about 50 % for patients in stage II patients and in a range of about 10 % to about 25 % for patients in stage III. However, since surgical resection and subsequent anticancer therapy such as chemotherapy reduce the quality of life of patients, such as weight loss, other treatment options are required.

Vaccine helps the body fight diseases by training the immune system to recognize and destroy harmful substances and diseased cells. Vaccines can be broadly divided into two types, i.e., prophylactic vaccines and therapeutic vaccines. A prophylactic vaccine is a traditional form of vaccines given to healthy people to prevent the development of certain diseases, whereas a therapeutic vaccine which is also called immunotherapy is given as a precautionary measure or to help stop a disease from growing and spreading in people diagnosed with the disease. A representative form of the therapeutic vaccine is a cancer vaccine.

A cancer vaccine may activate cytotoxic T cells so that the activated T cells can recognize and act against cancer cells that express a specific type of antigen, or a cancer vaccine may be designed to induce the production of antibodies that bind antigens present on the surface of cancer cells. The basic therapeutic goal of the cancer vaccine is to reduce the size of cancerous tissue that already exists in a cancer patient, or to suppress cancer recurrence after removing cancerous tissue from a cancer patient by surgical operation or chemotherapy.

Although extensive research on vaccines has been carried out over the past few years to treat cancer or prevent cancer recurrence, no successful cases have yet existed, except for sipuleucel-T (Provenge^{®}) which is a vaccine for prostate cancer. For causes of such a low success rate, it is described that antigens may be expressed at a low level depending on individuals although the antigens are specifically expressed in certain types of tumor cells, or that, in the case of metastatic tumors, antigen profiles between initially occurring tumor and metastatic tumor may change. Also, an immune evasion mechanism of tumor cells is also explained as a cause of the low success rate.

A preferred vaccine antigen should be expressed exclusively or at least at an increased level in tumor cells. HER2/neu (hereinafter referred to as 'HER2') is one of epidermal growth factor receptor (EGFR) family (EGFR2), and overexpression of EGFR is observed in many carcinomas. In particular, about 20 % of the entire patients with gastric cancer are known to be diagnosed with HER2-positive advanced gastric cancer.

HER2 is a transmembrane protein having an amino acid sequence of SEQ ID NO: 1, and consists of an intracellular domain (ICD, HER2 aa 676-1255) having protein tyrosine kinase activity, a transmembrane domain (TM, HER2 aa 653-675 ), and an extracellular domain (ECM, HER2 aa 22-652).

HER2 vaccines have been studied quite extensively in breast cancers, and several immunogenic peptides have been discovered. For example, in the case of a peptide of amino acids 369-377 of the HER2 protein (nelipepimut-S [NeuVax; HER2 aa369-377; E75 (NP-S)]), immunotherapy has been attempted in a way that the peptide is re-injected after loading into dendritic cells. However, no therapeutic or protective effects that are clinically significant were reported in women with advanced breast cancer. In addition, in the recent large-scale Phase III clinical trial, the clinical trial has been discontinued because there was no difference in disease-free survival (DFS) between a treatment group and a placebo group.

Other examples of the HER2 peptide vaccine include AE37 (LRMK-GVGSPYVSRLLGICL: LRMK-HER2 aa 776-790) which is a HER2-ICD peptide vaccine, GP2 (HER2 aa 654-662) which is a HER2-TM peptide vaccine, and the like. Some reports of the effect of increasing the survival rate with the vaccine have been reported. In addition, it has been reported in animal experiments that the vaccine against a HER2-ICD fragment (hereinafter referred to as 'HER2-ICD') reduces the size of the tumor compared to the vaccine against a HER2-ECD fragment (hereinafter referred to as 'HER2-ECD'). There has been a case of applying the HER2-ICD DNA vaccine to a patient with breast cancer patient. However, any result of applying the HER2-ICD DNA vaccine to gastric cancer rather than breast cancer has not been reported.

Therefore, in patients with gastric cancer, there is a need to develop the immune effect and therapeutic potential of the HER2-ICD DNA vaccine to generate a vaccine that will provide a reliable effect for the treatment or recurrence of gastric cancer.

### [Prior art documents]

### [Patent documents]

KR 10-1572474 (announcement date: November 30, 2015)
US 2002/0193329 (disclosed date: December 19, 2002)

### [Non-patent documents]

Breast Care 2016;11:116-121
Clin Cancer Res; 25(14) July 15, 2019

### DESCRIPTION OF EMBODIMENTS

### TECHNICAL PROBLEM

Accordingly, the present inventors conducted research to solve the above problem, and as a result, the present invention was completed by confirming that a DNA vaccine composition comprising a plasmid comprising a nucleic acid sequence encoding HER2-ICD can effectively inhibit tumor growth in a human gastric cancer cell line transplanted tumor mouse model.

An objective of the present invention is to provide a vaccine composition (hereinafter referred to as "HER2-ICD DNA vaccine composition") comprising a plasmid comprising a nucleic acid sequence encoding HER2-ICD.

An objective of the present invention is to provide a kit comprising the HER2-ICD DNA vaccine composition for treating gastric cancer.

An objective of the present invention is to provide a method of inhibiting progression and/or recurrence of gastric cancer by administering the vaccine composition or the kit to a subject.

An objective of the present invention is to provide use of the composition or the kit in the manufacture of a medical supply for treatment of gastric cancer.

### SOLUTION TO PROBLEM

To achieve the objectives above, the present invention provides a DNA vaccine composition comprising a plasmid comprising a nucleic acid sequence encoding HER2-ICD.

According to an embodiment of the present invention, the vaccine composition may be used in a group of patients with gastric cancer.

According to an embodiment of the present invention, the group of patients with gastric cancer may be a group of patients in which the HER2 protein is expressed.

According to one embodiment of the present invention, the vaccine composition may additionally include an adjuvant.

According to an embodiment of the present invention, the vaccine composition may be administered in combination with one or more anticancer agents selected from a chemical anticancer agent, a targeted anticancer agent, and an immune anticancer agent.

According to an embodiment of the present invention, the group of patients with gastric cancer may be a group of human patients.

### ADVANTAGEOUS EFFECTS OF DISCLOSURE

A vaccine composition according to the present invention is a vaccine composition comprising a plasmid comprising a nucleic acid sequence encoding HER2-ICD, and may effectively inhibit tumor growth in an animal model of gastric cancer, and thus may be usefully used in treatment of gastric cancer.

### BRIEF DESCRIPTION OF DRAWINGS

FIG. 1 shows a weight change for each individual in a xenograft mouse model transplanted with a gastric cancer cell line, when a HER2-ICD DNA vaccine (Vaccine), an anti-HER2 therapeutic antibody (Herceptin), and a combination thereof (Vaccine + Herceptin) are administered.
FIGS. 2 to 4 show inhibitory effects on tumor growth in a xenograft mouse model transplanted with gastric cancer cell line when a HER2-ICD DNA vaccine (Vaccine), an anti-HER2 therapeutic antibody (Herceptin), and a combination thereof (Vaccine + Herceptin) are administered.
FIGS. 5 to 7 show changes in spleen in a xenograft mouse model transplanted with a gastric cancer cell line, when a HER2-ICD DNA vaccine (Vaccine), an anti-HER2 therapeutic antibody (Herceptin), and a combination thereof (Vaccine + Herceptin) are administered.

### BEST MODE

Hereinafter, the present invention will be described in more detail.

In describing and claiming particular features of the present disclosure, the following terms will be used according to the definitions set forth below unless otherwise indicated.

It should be understood that, although certain aspects herein are described with the term "comprising," other similar aspects described in terms of "consisting of" and/or "consisting essentially of" are also provided.

The term "treat" refers to a result or a sign thereof that the tumor growth is inhibited or reduced when a cancer patient is examined by radiation or physical methods after the cancer patient is inoculated with a vaccine composition of the present invention. Moreover, the term also refers to a result or a sign thereof that the recurrence/relapse of cancer is prevented when the cancer patient is examined by radiation or physical methods after cancerous tissue of the cancer patient is removed by performing surgery or administering an anticancer drug to the cancer patient and then inoculated with a vaccine composition of the present invention.

The term "anticancer effect" refers to an effect of preventing or reducing the tumor growth when the vaccine composition of the present invention is administered to a cancer patient. Moreover, the term also refers to an effect of preventing the recurrence/relapse or growth of cancer after cancerous tissue of the cancer patient is removed by performing surgery or administering an anticancer drug to the cancer patient and then inoculated with a vaccine composition of the present invention.

The term "protective immunity" refers that the cancer progression or cancer recurrence/relapse is reduced in a subject in a way that the subject initiates an immune response against a HER2-ICD antigen and the immune system of the subject can target and destroy cells expressing the same antigen as the HER2-ICD antigen. In the present invention, the protective immunity is expressed as an anticancer effect that inhibits the tumor growth.

The term "anti-HER2 therapeutic antibody" refers to a monoclonal antibody capable of treating cancer by targeting an HER2 receptor and blocking proliferation of HER2-expressing tumor cells. Examples of the anti-HER2 therapeutic antibody includes trastuzumab (Herceptin^{®}), a biosimilar thereof, and the like, but are not limited thereto.

The term "booster" refers to a certain dose of an immunogen administered to a patient to enhance, prolong, or maintain the protective immunity and to overcome "down-regulation of T-cell responses" mediated by regulatory T-cells.

The term "pharmaceutically acceptable" refers to a substance that is acceptable to a patient in a pharmacological/toxicological point of view with respect to composition, formulation, safety, and the like. The term "pharmaceutically acceptable carrier" refers to a medium that does not interfere with an effect of biological activity of active ingredient(s), and the pharmaceutically acceptable carrier is non-toxic to a subject when administered.

The term "patient" or "subject" refers to a living organism in a condition in which a disease can be prevented or treated by administration of a vaccine composition of the present invention, such as a condition that suffers from, has been treated for, or is susceptible to gastric cancer, and may include both a human and an animal. Examples of the subject include mammals (e.g., a mouse, a monkey, a horse, a cow, a pig, a dog, a cat, etc.), but are not limited thereto, and the subject may preferably be a human. In addition, the term "patient" or "subject" in the present invention includes, without distinction, a patient with gastric cancer or a patient with gastric cancer in which HER2 is expressed.

The term "administering" refers to introducing a vaccine composition to a subject by using any of a variety of methods and delivery systems known in the art. Examples of administration routes include intravenous, intramuscular, subcutaneous, intraperitoneal, spinal, or other parenteral routes of administration, such as injection or infusion. The term "administration" used in the present specification refers to an intradermal administration way generally by injection, and may include, although not limited thereto, intravenous, intramuscular, intraarterial, intrathecal, or intralymphatic injection and infusion. The other parenteral routes may include topical, epithelial, or mucosal routes of administration, such as intranasal, vaginal, rectal, or sublingual routes. In the present specification, the terms "vaccine injection", "treatment", and "vaccination" are used without distinction in terms of acts of administering a vaccine to a subject.

In the present invention, the term "expressing HER2" refers to expressing HER2 gene or protein in cancer tissue. The expression level of the HER2 gene or protein does not distinguish between high expression and low expression.

In the present invention, the term "cancer immunotherapy drug" refers to a therapeutic drug that induces immune cells to selectively attack only cancer cells by stimulating the immune system, unlike existing anticancer drugs that attack cancer itself. In other words, it is an anticancer drug with a mechanism of restoring or strengthen the tumor recognition or destruction ability of the immune system to overcome the immunosuppression or immune evasion mechanism acquired by cancer cells. The cancer immunotherapy drug includes an immune checkpoint inhibitor, an immune cell therapeutic agent, and an immunoviral therapeutic agent, but are not limited thereto.

In the present invention, the term "immune checkpoint inhibitor" refers to a type of cancer immunotherapy drug that attacks cancer cells by blocking activation of immune checkpoint proteins involved in T cell inhibition and activating T cells, when some cancer cells evade immunity while utilizing the immune checkpoint of T cells that are immune cells in the body. Examples of the immune checkpoint inhibitor include a CTLA-4 inhibitor, a PD-1 inhibitor, a PD-L1 inhibitor, a LAG-3 inhibitor, a TIM-3 inhibitor, a TIGIT inhibitor, and a VISTA inhibitor, but are not limited thereto. In addition, the immune checkpoint inhibitor may be, as an antagonist, an antagonist antibody or a small molecule compound.

A composition disclosed in the present specification may be prepared as a DNA vaccine. In some embodiments of the present invention, a DNA vaccine may include a plasmid including a promoter, appropriate transcription and translation control factors, and a nucleic acid sequence encoding one or more polypeptides of the present invention. In some embodiments, the plasmid may additionally include enhancer sequences, for example, sequences that enhance expression levels, intracellular targeting, and the like.

In some embodiments of the present invention, the DNA vaccine may include a plasmid vector including a nucleic acid sequence encoding HER2-ICD of the present invention. In some embodiments, the plasmid may be a pUMVC3 plasmid (a conventional name is 'pNGVL3', and thus the two names may be used interchangeably). The nucleic acid sequence encoding HER2-ICD may have 100 % homology to the nucleic acid sequence of SEQ ID NO: 3, or in otherwise, may have 99 % or more, 95 % or more, 90 % or more, 85 % or more, 80 % or more homology to the nucleic acid sequence of SEQ ID NO: 3. However, in the case of having homology limited to the specific value above, the immunogenicity against HER2-ICD by vaccination may have the same or equivalent effect as a sequence having 100 % homology.

In some embodiments of the present invention, the human gastric cancer NCI-N87 cell line expressing HER2 protein may be transplanted into athymic nude mice to prepare xenograft gastric cancer mouse models, which may be then divided into 4 groups and treated with drugs as follows: a control group; a group treated with HER2-ICD DNA vaccine (Vaccine); a group treated with Herceptin as an anti-HER2 therapeutic antibody-treated group; and a group treated with HER2-ICD DNA vaccine and Herceptin in combination (Vaccine + Herceptin).

In all three drug-treated groups, the tumor growth was inhibited compared to the tumor growth in the control group (until Day 31 after the drug treatment). The group to which "HER2-ICD DNA vaccine" was administered alone showed the greatest inhibitory effect on the tumor growth, thereby showing more excellent results than the effect in the test group to which Herceptin was administered alone as well as in the test group to which Herceptin and "HER2-ICD DNA vaccine" were administered in combination.

Regarding HER2-expressing advanced gastric cancer, in consideration that HER2-direct targeting therapy using an anti-HER2 therapeutic antibody or combined therapy with anti-HER2 therapeutic antibody and chemotherapy (a combination of 5-fluorouracil and cisplatin or a combination of capecitabine and oxaliplatin) is a common treatment, the fact that the administration of the HER2-ICD DNA vaccine of the present invention alone exhibits a better inhibitory effect on the tumor growth than the administration of Herceptin alone can be seen as suggesting a new possibility in the treatment of HER2 expression gastric cancer.

In particular, the safety of the HER2-ICD DNA vaccine of the present invention has already been confirmed in the phase 1 clinical trial (NCT00436254), and thus the DNA vaccine treatment of the present invention may be applied as a treatment that can reduce the burden on patients compared to the existing treatment.

In some embodiments of the present invention, the DNA vaccine may additionally encode immunomodulatory molecules that modulate the resulting immune response, such as enhancing the efficacy of the vaccine, stimulating the immune system, or reducing the immunologic inhibition.

Since the vaccine composition of the present invention aims to induce and/or maintain a therapeutic effect on the growth or recurrence of gastric cancer, the vaccine composition of the present invention may be administered to a patient according to any schedule suitable for inducing and/or maintaining a cytotoxic T cell response to HER2-ICD. For example, after the vaccine composition as described and exemplified herein is administered as a primary vaccination to a patient on schedule, a booster may be administered to induce and/or maintain the protective immunity.

In some embodiments of the present invention, the vaccine composition may be administered to a patient once a month, twice a month, or more times a month. After finishing the vaccination schedule, the booster may be administered once or multiple times at regular intervals, for example, once or multiple times at intervals of 1 month or once or multiple times with a period of 6 months or more. In some embodiments of the present invention, the HER2-ICD vaccine may be administered three times a month as primary vaccination, and an additional booster may be administered after 6 months or 1 year.

Here, the booster may be used in the same dose and may have the same composition as the vaccine composition used for the primary vaccination, or the dose and/or composition of the booster may vary.

The vaccine composition described in the present specification may include, in addition to the plasmid including DNA encoding HER2-ICD, a pharmaceutically acceptable excipient, carrier, diluent, buffer, stabilizer, preservative, adjuvant, other substances well known in the art. These substances may be non-toxic, and may not interfere with the pharmaceutical activity of active ingredient(s). The pharmaceutical carrier or diluent may be, for example, an aqueous solution. The carrier or other substances that may be included in the vaccine composition of the present invention may be selected differently depending on the route of administration (e.g., oral, intravenous, dermal or subcutaneous, nasal, intramuscular, intradermal, and intraperitoneal administration routes).

The vaccine composition of the present invention may include one or more "pharmaceutically acceptable carriers". Typical examples of the pharmaceutically acceptable carriers include proteins, saccharides, polylactic acids, polyglycolic acids, polymeric amino acids, amino acid copolymers, sucrose, and trehalose, and the like. Such carriers are well known in the art. The vaccine composition may also include a diluent such as water, saline, glycerol, and the like. In addition, auxiliary substances such as a wetting agent, an emulsifying agent, a pH buffer substance, and the like may be present in the vaccine composition. Sterile and pyrogen-free phosphate-buffered saline (PBS) and tromethamine (Tris) are representative carriers.

An aspect of the present invention relates to a vaccine composition or kit as described above, including one or more plasmid DNAs as active ingredients. The kit of the present invention may include the vaccine composition of the present invention, an instruction for using the vaccine composition, and the like.

The vaccine composition or kit of the present invention may be for use in a method of inducing an immune response or providing treatment, vaccination, or immunotherapy in a subject, and the vaccine composition may be a vaccine or an immunotherapeutic composition. Such treatment or vaccination may include administration of the vaccine composition of the present invention to a subject. The vaccine composition may be administered to a subject on a fixed schedule.

The vaccine composition or kit of the present invention may be used for the treatment of gastric cancer in a subject. The subject may be a human, and the gastric cancer may be a gastric cancer expressing a HER2 protein.

The vaccine composition of the present invention may be lyophilized or may be in an aqueous form, such as an aqueous solution or a suspension. Liquid formulations of such a type may be ideal for injection as being able to be administered directly in a packaged form without the need for reconstituting the composition in an aqueous medium. The vaccine composition may be provided in vials, or may be provided in a pre-filled syringe. A syringe may be supplied with or without a needle. The syringe may contain a single dose, whereas the vial may contain a single dose or multiple doses. In addition, the vaccine composition of the present invention may be administered by using a microneedle.

In addition, the vaccine composition of the present invention may be formulated as a delayed release vehicle or a depot preparation. Such long-acting formulations may be administered by inoculation or implantation (for example, subcutaneously or intramuscularly) or by injection. Thus, for example, the vaccine composition may be formulated with a suitable polymeric or hydrophobic material (e.g., an emulsion in acceptable oil) or an ion-exchange resin, or may be formulated as a water poorly-soluble derivative (e.g., a water poorly-soluble salt). Liposomes and emulsions are well-known examples as delivery vehicles suitable for use as carriers.

The vaccine composition of the present invention may include an antimicrobial agent when packaged in a multiple-dose format. The antimicrobial agent may be selected from 2-phenoxyethanol or parabens (e.g., methyl, ethyl, or propyl parabens). An optional preservative may be preferably present at a low level. The preservative may be added exogenously and/or may be a component of bulk antigen that is mixed to form the composition.

The vaccine composition of the present invention may be provided in the form of kit including an instruction and the like.

In addition, the kit of the present invention may include the vaccine composition of the present invention, the instruction, and the like, but may not include the anti-HER2 therapeutic antibody.

The vaccine composition of the present invention may include one or more adjuvants to increase the immunogenicity of the composition.

In the present invention, the composition may additionally include an anti-cancer adjuvant. More preferably, an example of the anti-cancer adjuvant includes a STING (Stimulator of Interferon Gene) agonist, but is not limited thereto.

The suitable adjuvant may include an aluminum salt, such as aluminum hydroxide or aluminum phosphate, but may be a salt of calcium, iron, or zinc. Alternatively, the suitable adjuvant may be an insoluble suspension of acylated tyrosine or acylated saccharide, or may be selected from cationically- or anionically induced saccharides and the like, but is not limited thereto. In addition, the adjuvant may include a cytokine that is used as an immunomodulatory agent.

The cytokine suitable for use as the immunomodulatory agent may be selected from an interleukin (e.g., IL-1, IL-2, IL-4, IL-5, IL-6, IL-7, IL-12, etc.); a M-CSF (macrophage colony stimulating factor), a TNF (tumor necrosis factor), a GM-CSF (granulocyte macrophage colony stimulating factor), and the like, but is not limited thereto.

The vaccine composition of the present invention may be administered in combination with one or more anticancer agents selected from a chemical anticancer agent, a targeted anticancer agent, and an immune anticancer agent. The one or more anticancer agents selected from a chemical anticancer agent, a targeted anticancer agent, and an immune anticancer agent that can be administered in combination with the vaccine composition of the present invention may be administered simultaneously or sequentially with a time gap, and may be selected depending on an appropriate time and cycle.

In the present invention, the chemical anticancer agent may be also called a cytotoxic anticancer agent or a chemical anticancer agent. The chemical anticancer agent may be, for example, gemcitabine, cytarabine, carboplatin (Paraplatin), cisplatin (Platinol, Platinol-AQ), Crizotinib (Zalkori), cyclophosphamide (Cytoxane, Neosar), docetaxel (Taxotere), doxorubicin (Adriamycin), etoposide (BePesid), fluorouracil (5-fluorouracil, 5-FU), irinotecan (Camptosar), liposome-encapsulated doxorubicin (Doxyl), methotrexate (Polex, Mexate, Amethopterin), paclitaxel (Taxol, Abraxane), topotecan (Hycamtin), trabectedin (Yondelis), vincristine (Oncobin, Vincasar PFS), vinbrastine (Velban), capecitabine, oxaliplatin, and the like, but is not limited thereto.

In the present invention, the targeted anticancer agent may be one or more targeted anticancer agent selected from the group consisting of trametinib, vemurafenib, alpelisib, dactolisib, gefitinib, erlotinib, lapatinib, sunitinib, sorafenib, crizotinib, dabrafenib, trastuzumab, cetuximab, bevacizumab, panitumumab, ipilimumab, pertuzumab, tofacitinib, imatinib, bortezomib, ofatumumab, and alemtuzumab, but is not limited thereto.

The targeted anticancer agent may include an antibody-drug conjugate (ADC) as a next-generation targeted anticancer agent.

In the present invention, the immunocancer agent may be an immune checkpoint inhibitor for any one selected from the group consisting of CTLA-4 (cytotoxic T lymphocyte-associated protein 4), PD-1 (programmed cell death protein 1), PD-L1 (programmed death ligand 1), LAG3 (lymphocyte activation gene-3), TIM-3 (T-cell immunoglobulin and mucin-domain containing-3), TIGIT (T-cell Immunoreceptor with IG and ITIM domain, and VISTA (V domain Ig suppressor of T cell activation), but is not limited thereto.

In some embodiments of the present invention, the administration of the HER2-ICD DNA vaccine composition may be performed simultaneously with treatment with other cancer therapeutic agents. For the cancer therapeutic agent, standard substances, such as fluorouracil, doxorubicin, paclitaxel, and the like may be used, but is not limited thereto.

Unless otherwise indicated, all numbers used in the present specification and claims should be understood as, whether recited or not, being modifiable in all instances by the term "about". In addition, the precise numbers used in the present specification and claims should be understood to form further embodiments of the present disclosure. Efforts have been made to ensure the accuracy of the numerical values disclosed in Examples below. However, all measured values may include implicitly certain error values generated from the standard deviations measured by respective measurement techniques.

### MODE OF DISCLOSURE

Hereinafter, the present invention will be described in more detail through Examples below. Examples below are only for illustrating the present invention in more detail, and it will be apparent to those skilled in the art that the scope of the present invention is not limited by Examples below according to the gist of the present invention.

### Examples

Anti-tumor efficacy of the HER2-ICD DNA vaccine was measured by administering the HER2-ICD DNA vaccine alone or in combination with Herceptin (anti-HER2 antibody). A xenograft mouse model in which an athymic mouse was transplanted with NCI-N87 (ATCC CRL-2855), which is a human-derived gastric cancer cell line expressing HER2 protein, was used as an example of an animal model for gastric cancer.

All animal tests were ethically conducted under the approval of the Institutional Animal Care and Use Committee (IACUC) of Laboratory animal facility of Osong Medical Innovation Foundation (KBIO) (KBIO-IACUC-2020-120).

### Preparation of xenograft mouse model of gastric cancer

Athymic-nude mice (athymic-NCr-nu strain) weighing about 16 g to 18 g and aged about 5 weeks were purchased from KOATECH, and then acclimated for at least 1 week.

5×10⁶ cells of the human-derived gastric cancer cell line NCI-N87 were suspended in 200 µl PBS of per individual, and then injected subcutaneously into the axillary region between the scapula and chest wall on the right side of the mouse.

To analyze the anti-tumor effect of the HER2-ICD DNA vaccine, 24 mice of which the tumor size reached 150 mm³ (average tumor size of 150.5t16.4 mm³) on Day 11 after the cell transplantation were selected from among the mice transplanted with the NCI-N87 cells, and six mice were assigned to each group.

### Vaccine formulation

A DNA sequence (SEQ ID NO: 3) encoding HER2-ICD was inserted into a pNGVL3 (pUMVC3) plasmid vector and confirmed by DNA sequencing.

The plasmid DNA vaccine, pNGVL3-hICD, was amplified and quantified under GMP conditions, and then prepared in vials. Single-dose vials contained plasmid DNA (pNGVL3-HER2 ICD) suspended in tromethamine/EDTA as a stabilizing buffer (1.2 ml). The vials were produced by VGXI, Inc., USA and stored at -20 °C by using a master cell bank (MCB) according to the US Pharmacopoeia standard after undergoing microbiological, sterility, and stability tests.

To each mouse, the vaccine formulation was diluted with PBS and administered at a final dose of 5 mg/kg (100 µg/20 g/150 µl). Herceptin (Roche) was administered at a dose of 40 µg/20 g/100 µl after being dissolved in physiological saline.

### Drug administration and administration schedule

The HER2-ICD DNA vaccine was administered intradermally, and Herceptin was administered intraperitoneally.

To mice that had already been transplanted with the NCI-N87 cells and confirmed for the tumor development, the HER2-ICD DNA vaccine was administered intradermally three times on Days 0, 7, and 14, and Herceptin was administered intraperitoneally 5 times on Days 0, 7, 14, 21, and 28.

As a control group, PBS was administered to the mice.

### Observation and inspection entry

All mice were observed for general symptoms once a day.

During the test period, the mice were weighed 14 times in total (on Days 0, 3, 5, 7, 10, 12, 14, 17, 19, 21, 24, 26, 28, and 31), and changes in body weight was observed until Day 31 based on the body weight (100 %) on the start date of administration.

The tumor size and the inhibitory rate on tumor growth were measured 8 times in total from Day 8 of cancer cell transplantation to Day 32 (on Days 8, 11, 15, 18, 22, 25, 30, and 32) in three directions using a vernier caliper for each individual, and the tumor size was calculated. Starting from the point where the tumor size became 150 mm³ on Day 11 of the cancer cell transplantation (Day 0), the tumor size for each individual was calculated 14 times in total (Days 0, 3, 5, 7, 10 12, 14, 17, 19, 21, 24, 26, 28, and 31) and compared with the control group in each test group to calculate a rate of change in the tumor growth.

### Autopsy and inspection entry

On the last day of the test, an anesthetic (a mixture of 50 mg/kg of Zoletil50 and 10 mg/kg of Rompun) was injected intramuscularly into the mice to induce euthanasia under anesthesia-induced state after scarification. Then, the tumor and spleen were excised, weighed, and photographed.

### Analysis of anti-tumor effect of HER2-ICD DNA vaccine

No animals died in any of the test groups during the test, and no abnormal symptoms were observed when the test substances were administered alone or in combination.

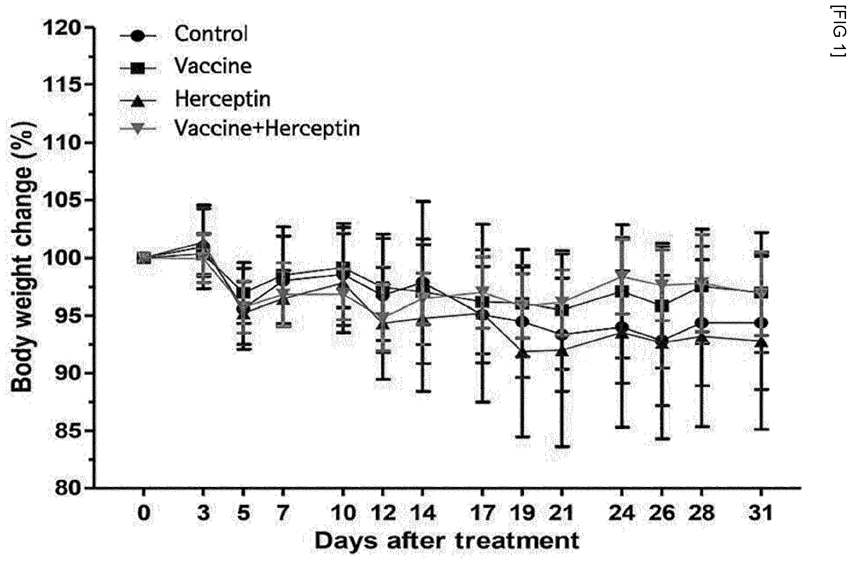

As a result of observing the change in body weight during the test period, a tendency for weight loss appeared in a group Herceptin treatment group and a group treated with both Herceptin and HER2-ICD DNA vaccine (hereinafter referred to as a Vaccine+Herceptin treatment group) compared to the control group (PBS treatment group). A tendency for gradual weight loss appeared from Day 5 to the last day (Day 31) in all test groups, but there was no statistical significance. The body weights on the last day were observed to be 94.4±5.8 % in the control group, 97.0±5.2 % in a group treated with the HER2 ICD DNA vaccine (hereinafter referred to as a 'Vaccine treatment group'), 92.8±7.7 % in the Herceptin treatment group, and 96.9±3.6 % in the Vaccine+Herceptin treatment group, respectively (FIG. 1).

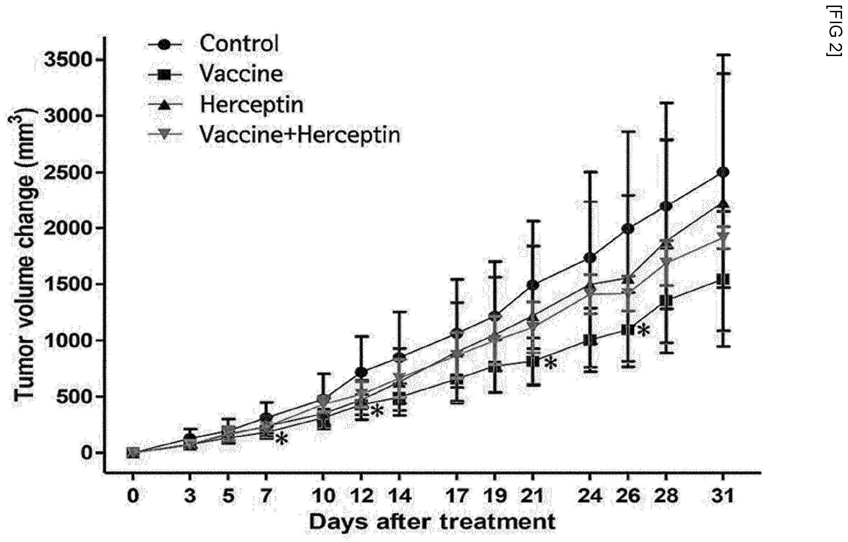

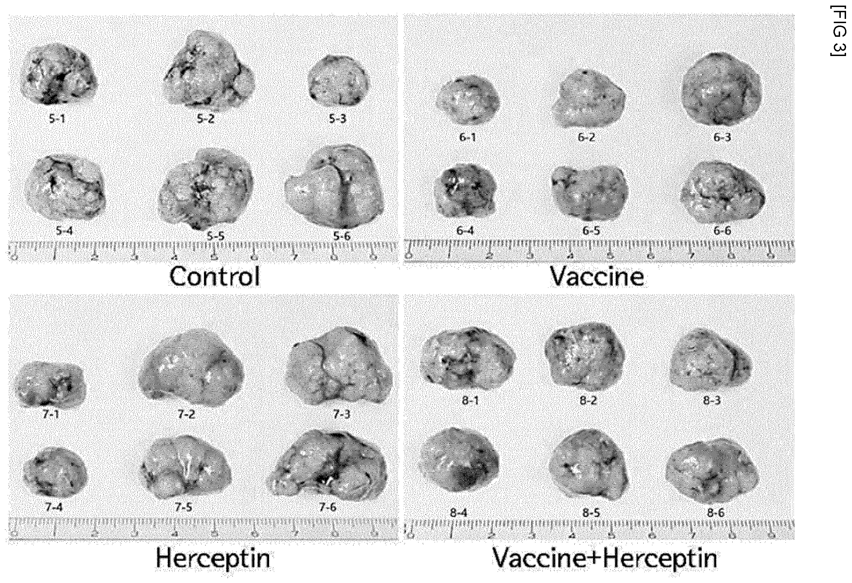

As a result of measuring the rate of size change in the tumor growth, the tumor growth inhibition was continuously observed from Day 3 to the last day in all of the Vaccine treatment group, the Herceptin treatment group, and the Vaccine+Herceptin treatment group, compared to the control group. In particular, at some points (Days 7, 12, 21, and 26) with the Vaccine treatment group, the inhibitory effect on the tumor growth recognized with a statistically significance was shown (p<0.05) (FIG. 2). The tumors extracted from each mouse are shown in FIG. 3.

Therefore, it was confirmed that the HER2-ICD vaccine was able to inhibit the tumor growth in the xenograft gastric cancer mouse model by inducing a protective immune response therein.

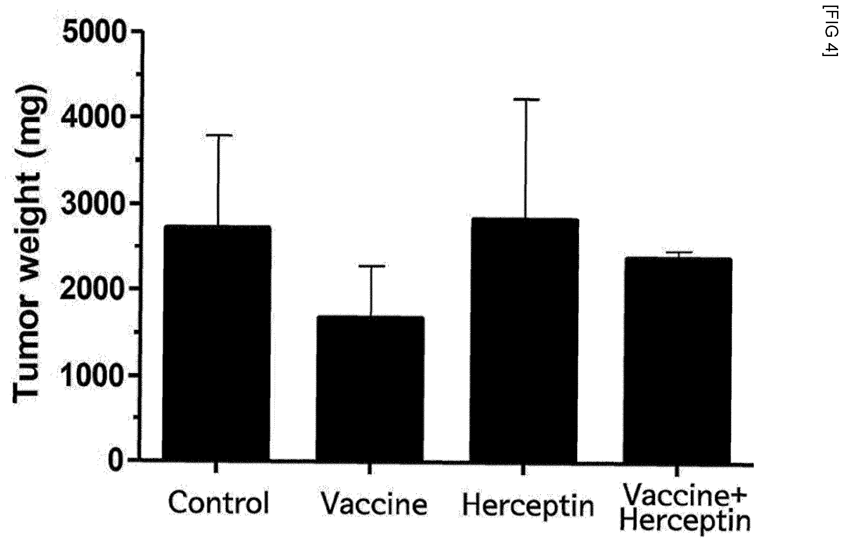

As a result of weighing the tumor, a decrease in the tumor weight was observed in the Vaccine treatment group and the Vaccine+Herceptin treatment group, compared to the control group. However, no statistical significance was observed with respect to the decrease in the tumor weight (FIG. 4).

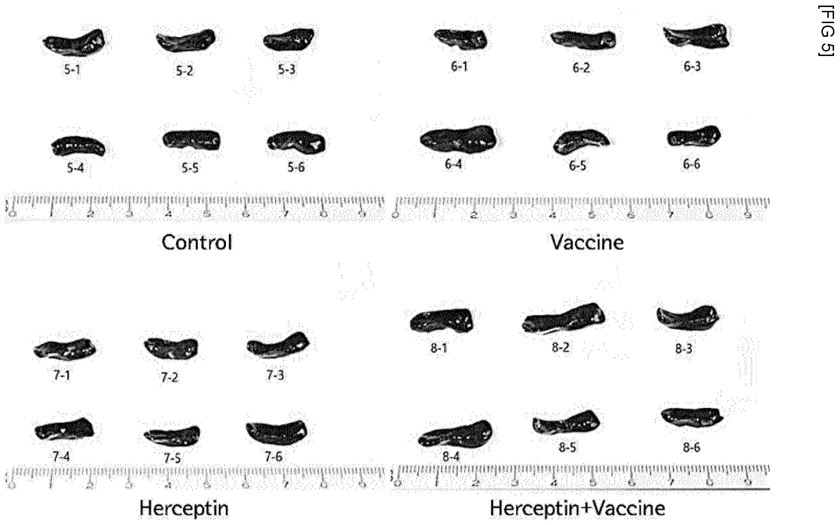

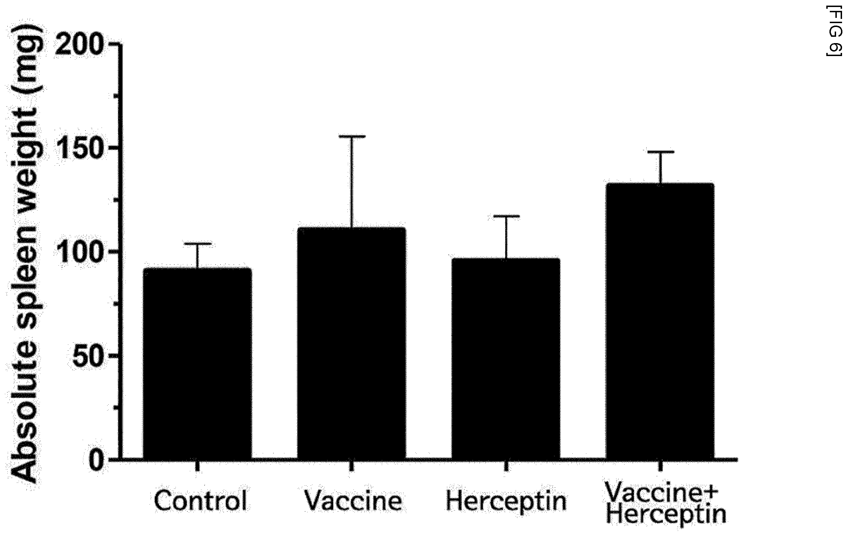

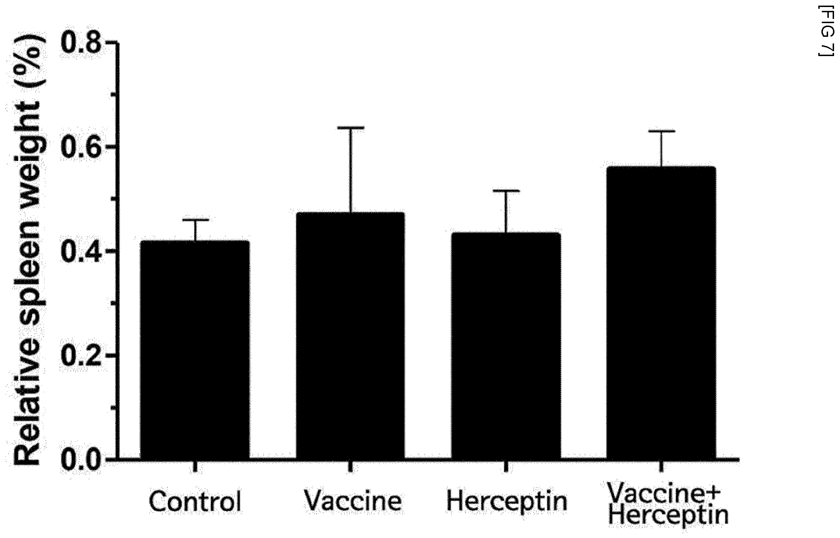

As a result of examining the spleen with naked eyes and weighing the spleen, it was observed that the size of the spleen increased in the Vaccine treatment group and the Vaccine+Herceptin treatment group, compared to the control group (FIG. 5), and that the spleen weight in terms of both absolute weight and relative weight, was high in the Vaccine treatment group, Herceptin treatment group and the Vaccine+Herceptin treatment group, compared to the control group. In particular, the highest spleen weight was observed in the Vaccine+Herceptin treatment group, but no statistical significance was confirmed (FIGS. 6 and 7).

Considering the results above overall, no abnormal symptoms due to administration of the HER2-ICD DNA vaccine alone or co-administration of the HER2-ICD DNA vaccine and Herceptin to the animal models transplanted with NCI-N87 gastric cancer cells were observed.

In addition, according to analysis of the anti-tumor effect in the animal transplanted with the NCI-N87 gastric cancer cells, the anti-tumor effect that inhibits the tumor growth was observed by the administration of the HER2-ICD DNA vaccine alone and the co-administration of the HER2-ICD DNA vaccine and Herceptin, rather than by the administration of Herceptin alone. In particular, the anti-tumor effect was shown more dominantly in the case where the HER2-ICD DNA vaccine was administered alone than in the cases where Herceptin alone was administered or HER2-ICD DNA vaccine and Herceptin were administered in combination. Therefore, the present invention suggests the possibility that use of HER2-ICD DNA vaccine alone can effectively treat gastric cancer in the treatment of HER2 protein-expressing gastric cancer.

### Sequence List Text

SEQ ID NO: 1 (HER2 full-length sequence) 
MELAALCRWGLLLALLPPGAASTQVCTGTDMKLRLPASPETHLDMLRHLYQGCQVVQGNLELTYLPTNASLSFLQDI
QEVQGYVLIAHNQVRQVPLQRLRIVRGTQLFEDNYALAVLDNGDPLNNTTPVTGASPGGLRELQLRSLTEILKGGVL
IQRNPQLCYQDTILWKDIFHKNNQLALTLIDTNRSRACHPCSPMCKGSRCWGESSEDCQSLTRTVCAGGCARCKGPL
PTDCCHEQCAAGCTGPKHSDCLACLHFNHSGICELHCPALVTYNTDTFESMPNPEGRYTFGASVTACPYNYLSTDVG
SCTLVCPLHNQEVTAEDGTQRCEKCSKPCARVCYGLGMEHLREVRAVTSANIQEFAGCKKIFGSLAFLPESFDGDPA
SNTAPLQPEQLQVFETLEEITGYLYISAWPDSLPDLSVFQNLQVIRGRILHNGAYSLTLQGLGISWLGLRSLRELGS
GLALIHHNTHLCFVHTVPWDQLFRNPHQALLHTANRPEDECVGEGLACHQLCARGHCWGPGPTQCVNCSQFLRGQEC
VEECRVLQGLPREYVNARHCLPCHPECQPQNGSVTCFGPEADQCVACAHYKDPPFCVARCPSGVKPDLSYMPIWKFP
DEEGACQPCPINCTHSCVDLDDKGCPAEQRASPLTSIISAVVGILLVVVLGVVFGILIKRRQQKIRKYTMRRLLQET
ELVEPLTPSGAMPNQAQMRILKETELRKVKVLGSGAFGTVYKGIWIPDGENVKIPVAIKVLRENTSPKANKEILDEA
YVMAGVGSPYVSRLLGICLTSTVQLVTQLMPYGCLLDHVRENRGRLGSQDLLNWCMQIAKGMSYLEDVRLVHRDLAA
RNVLVKSPNHVKITDFGLARLLDIDETEYHADGGKVPIKWMALESILRRRFTHQSDVWSYGVTVWELMTFGAKPYDG
IPAREIPDLLEKGERLPQPPICTIDVYMIMVKCWMIDSECRPRFRELVSEFSRMARDPQRFVVIQNEDLGPASPLDS
TFYRSLLEDDDMGDLVDAEEYLVPQQGFFCPDPAPGAGGMVHHRHRSSSTRSGGGDLTLGLEPSEEEAPRSPLAPSE
GAGSDVFDGDLGMGAAKGLQSLPTHDPSPLQRYSEDPTVPLPSETDGYVAPLTCSPQPEYVNQPDVRPQPPSPREGP
LPAARPAGATLERPKTLSPGKNGVVKDVFAFGGAVENPEYLTPQGGAAPQPHPPPAFSPAFDNLYYWDQDPPERGAP
PSTFKGTPTAENPEYLGLDVPV
SEQ ID NO: 2 (HER2-ICD amino acid sequence) 
MKRRQQKIRKYTMRRLLQETELVEPLTPSGAMPNQAQMRILKETELRKVKVLGSGAFGTVYKGIWIPDGENVKIPVA
IKVLRENTSPKANKEILDEAYVMAGVGSPYVSRLLGICLTSTVQLVTQLMPYGCLLDHVRENRGRLGSQDLLNWCMQ
IAKGMSYLEDVRLVHRDLAARNVLVKSPNHVKITDFGLARLLDIDETEYHADGGKVPIKWMALESILRRRFTHQSDV
WSYGVTVWELMTFGAKPYDGIPAREIPDLLEKGERLPQPPICTIDVYMIMVKCWMIDSECRPRFRELVSEFSRMARD
PQRFVVIQNEDLGPASPLDSTFYRSLLEDDDMGDLVDAEEYLVPQQGFFCPDPAPGAGGMVHHRHRSSSTRSGGGDL
TLGLEPSEEEAPRSPLAPSEGAGSDVFDGDLGMGAAKGLQSLPTHDPSPLQRYSEDPTVPLPSETDGYVAPLTCSPQ
PEYVNQPDVRPQPPSPREGPLPAARPAGATLERAKTLSPGKNGVVKDVFAFGGAVENPEYLTPQGGAAPQPHPPPAF
SPAFDNLYYWDQDPPERGAPPSTFKGTPTAENPEYLGLDVPV
SEQ ID NO: 3 (HER2-ICD nucleotide sequence)

ATGAAGCGACGGCAGCAGAAGATCCGGAAGTACACGATGCGGAGACTGCTGCAGGAAACGGAGCTGGTGGAGCCGCT
GACACCTAGCGGAGCGATGCCCAACCAGGCGCAGATGCGGATCCTGAAAGAGACGGAGCTGAGGAAGGTGAAGGTGC
TTGGATCTGGCGCTTTTGGCACAGTCTACAAGGGCATCTGGATCCCTGATGGGGAGAATGTGAAAATTCCAGTGGCC
ATCAAAGTGTTGAGGGAAAACACATCCCCCAAAGCCAACAAAGAAATCTTAGACGAAGCATACGTGATGGCTGGTGT
GGGCTCCCCATATGTCTCCCGCCTTCTGGGCATCTGCCTGACATCCACGGTGCAGCTGGTGACACAGCTTATGCCCT
ATGGCTGCCTCTTAGACCATGTCCGGGAAAACCGCGGACGCCTGGGCTCCCAGGACCTGCTGAACTGGTGTATGCAG
ATTGCCAAGGGGATGAGCTACCTGGAGGATGTGCGGCTCGTACACAGGGACTTGGCCGCTCGGAACGTGCTGGTCAA
GAGTCCCAACCATGTCAAAATTACAGACTTCGGGCTGGCTCGGCTGCTGGACATTGACGAGACAGAGTACCATGCAG
ATGGGGGCAAGGTGCCCATCAAGTGGATGGCGCTGGAGTCCATTCTCCGCCGGCGGTTCACCCACCAGAGTGATGTG
TGGAGTTATGGTGTGACTGTGTGGGAGCTGATGACTTTTGGGGCCAAACCTTACGATGGGATCCCAGCCCGGGAGAT
CCCTGACCTGCTGGAAAAGGGGGAGCGGCTGCCCCAGCCCCCCATCTGCACCATTGATGTCTACATGATCATGGTCA
AATGTTGGATGATTGACTCTGAATGTCGGCCAAGATTCCGGGAGTTGGTGTCTGAATTCTCCCGCATGGCCAGGGAC
CCCCAGCGCTTTGTGGTCATCCAGAATGAGGACTTGGGCCCAGCCAGTCCCTTGGACAGCACCTTCTACCGCTCACT
GCTGGAGGACGATGACATGGGGGACCTGGTGGATGCTGAGGAGTATCTGGTACCCCAGCAGGGCTTCTTCTGTCCAG
ACCCTGCCCCGGGCGCTGGGGGCATGGTCCACCACAGGCACCGCAGCTCATCTACCAGGAGTGGCGGTGGGGACCTG
ACACTAGGGCTGGAGCCCTCTGAAGAGGAGGCCCCCAGGTCTCCACTGGCACCCTCCGAAGGGGCTGGCTCCGATGT
ATTTGATGGTGACCTGGGAATGGGGGCAGCCAAGGGGCTGCAAAGCCTCCCCACACATGACCCCAGCCCTCTACAGC
GGTACAGTGAGGACCCCACAGTACCCCTGCCCTCTGAGACTGATGGCTACGTTGCCCCCCTGACCTGCAGCCCCCAG
CCTGAATATGTGAACCAGCCAGATGTTCGGCCCCAGCCCCCTTCGCCCCGAGAGGGCCCTCTGCCTGCTGCCCGACC
TGCTGGTGCCACTCTGGAAAGGGCCAAGACTCTCTCCCCAGGGAAGAATGGGGTCGTCAAAGACGTTTTTGCCTTTG
GGGGTGCCGTGGAGAACCCCGAGTACTTGACACCCCAGGGAGGAGCTGCCCCTCAGCCCCACCCTCCTCCTGCCTTC
AGCCCAGCCTTCGACAACCTCTATTACTGGGACCAGGACCCACCAGAGCGGGGGGCTCCACCCAGCACCTTCAAAGG
GACACCTACGGCAGAGAACCCAGAGTACCTGGGTCTGGACGTGCCAGTGTAA

## Claims

1. A vaccine composition for inducing an anticancer effect in a subject, the vaccine composition comprising a pharmaceutically acceptable carrier and a plasmid vector comprising a nucleotide sequence encoding an HER2-ICD region having SEQ ID NO: 2 or 80% or more sequence homology thereto.

2. The vaccine composition of claim 1, wherein the nucleotide sequence has SEQ ID NO: 3 or 80 % or more sequence homology thereto.

3. The vaccine composition of claim 1 or claim 2, wherein the subject is a gastric cancer patient in which HER2 protein is expressed.

4. The vaccine composition of claim 3, wherein the subject is a human.

5. The vaccine composition of claim 4, wherein an anti-HER2 therapeutic antibody is not administered in combination with the vaccine composition to the subject.

6. The vaccine composition of claim 1 or claim 2, further comprising an adjuvant.

7. The vaccine composition of claim 1 or claim 2, wherein the vaccine composition is administered by injection or inoculation.

8. The vaccine composition of claim 7, wherein the composition is administered by intradermal injection.

9. A kit for treating gastric cancer expressing HER2, the kit comprising the vaccine composition of claim 1 or claim 2 and an instruction.

10. The kit of claim 9, wherein the kit does not comprise an anti-HER2 therapeutic antibody.
